# EUROPEAN PATENT SPECIFICATION

(11) **EP 4 104 800 B1**
(45) Date of publication and mention of the grant of the patent: **29.01.2025**
(21) Application number: 22178968.8
(22) Date of filing: 14.06.2022
(51) Int. Cl.: A61F 2/60, A61F 2/64, A61F 2/74

(54) **ARTIFICIAL KNEE JOINT, METHOD OF CONTROLLING ARTIFICIAL KNEE JOINT, AND PROGRAM TO CONTROL ARTIFICIAL KNEE JOINT**
KÜNSTLICHES KNIEGELENK, VERFAHREN ZUM STEUERN EINES KÜNSTLICHEN KNIEGELENKS UND PROGRAMM ZUM STEUERN EINES KÜNSTLICHEN KNIEGELENKS
ARTICULATION DU GENOU ARTIFICIELLE, PROCÉDÉ DE COMMANDE DE L'ARTICULATION ARTIFICIELLE DU GENOU ET PROGRAMME DE COMMANDE DE L'ARTICULATION ARTIFICIELLE DU GENOU

(30) Priority: 14.06.2021 JP 2021098688
(43) Date of publication of application: 21.12.2022
(73) Proprietor: Nabtesco Corporation, Tokyo 102-0093 (JP)
(72) Inventor: Hashimoto, Hiroaki, Tokyo, 102-0093 (JP)
(74) Representative: Grünecker Patent- und Rechtsanwälte PartG mbB

(56) References cited:
- US-A1- 2003 125 814
- US-A1- 2008 288 086
- US-A1- 2010 191 347

## Description

The present invention relates to an artificial knee joint.

An artificial knee joint or a prosthetic limb worn by a person who has lost a leg due to an injury or a disease, in which the rotational resistance of the knee shaft is controlled in accordance with the phase of walking of the wearer, is known. In the Stance phase in which the prosthetic limb is in contact with the ground and is under load, the rotational resistance of the knee shaft is increased to prevent the knee from being bent under load. In the Swing phase in which the prosthetic limb leaves the ground and is swung, the rotational resistance of the knee shaft is decreased to bend the knee and prevent the prosthetic limb from touching the ground.
US 2003/125 814 A1 discloses an artificial knee joint according to the preamble of claim 1.
[Patent literature 1] JP2011-101807

We have studied multiple aspects of control of the rotational resistance of a knee shaft for the purpose of further improving the convenience of an artificial knee joint or a prosthetic limb.

The present invention addresses the above-described issue and a purpose thereof is to provide a highly convenient artificial knee joint or prosthetic limb.

This is achieved by an artificial knee joint according to claim 1. Preferred embodiments are laid down in the dependent claims. An artificial knee joint according to an aspect disclosed herein includes: a thigh joint part provided on a side of a thigh part; a lower leg part coupled to the thigh joint part and provided to be rotatable around a knee shaft; a thigh part inclination angle acquisition unit that acquires an inclination angle formed by the thigh part relative to a vertical line passing through the knee shaft; and a rotational resistance control unit that weakens a rotational resistance of the knee shaft in accordance with a transition from a positive inclination angle formed when the thigh part is inclined rearward relative to the vertical line to a negative inclination angle formed when the thigh part is inclined forward relative to the vertical line.

Another aspect disclosed herein also relates to an artificial knee joint. The artificial knee joint is provided with a thigh joint part provided on a side of a thigh part and with a lower leg part coupled to the thigh joint part and provided to be rotatable around a knee shaft, and includes: a forward movement amount sensing unit that senses a forward movement amount of a user wearing the artificial knee joint; and a rotational resistance control unit that strengthens a rotational resistance of the knee shaft when the forward movement amount sensed makes a transition from a state in which the forward movement amount is equal to or larger than a predetermined forward movement amount threshold value to a state in which forward movement amount is smaller than the forward movement amount threshold value.

Another aspect disclosed herein also relates to an artificial knee joint. The artificial knee joint is provided with a thigh joint part provided on a side of a thigh part and with a lower leg part coupled to the thigh joint part and provided to be rotatable around a knee shaft, and includes: a forward movement amount sensing unit that senses a forward movement amount of a user wearing the artificial knee joint; and a rotational resistance control unit that enables controlling a rotational resistance of the knee shaft to be weaker when the forward movement amount sensed is equal to or greater than a predetermined forward movement amount threshold value.

Another aspect disclosed herein also relates to an artificial knee joint. The artificial knee joint is provided with a thigh joint part provided on a side of a thigh part and with a lower leg part coupled to the thigh joint part and provided to be rotatable around a knee shaft, and includes: a rotational resistance control unit that weakens a rotational resistance of the knee shaft in accordance with a transition of a user walking and wearing the artificial knee joint from a Stance phase to a Swing phase; a walk information acquisition unit that acquires walk information indicating a state of walk of the user; and a control timing determination unit that determines a timing according to which the rotational resistance control unit weakens the rotational resistance of the knee shaft, based on the walk information.

Another aspect disclosed herein relates to an adjustment support apparatus for an artificial knee joint. The apparatus is an adjustment support apparatus for an artificial knee joint provided with a thigh joint part provided on a side of a thigh part and with a lower leg part coupled to the thigh joint part and provided to be rotatable around a knee shaft, the adjustment support apparatus including: a walk information acquisition unit that acquires walk information indicating a state of walk of a user walking and wearing the artificial knee joint; and a control timing determination unit that determines a timing according to which a rotational resistance of the knee shaft is weakened in accordance with a transition of the artificial knee joint from a Stance phase to a Swing phase, based on the walk information.

Optional combinations of the aforementioned constituting elements, and implementations of the disclosure in the form of methods, apparatuses, systems, recording mediums, and computer programs may also be practiced as additional modes of the present invention.

Embodiments will now be described by way of examples only, with reference to the accompanying drawings which are meant to be exemplary, not limiting and wherein like elements are numbered alike in several Figures in which:
Fig. 1 shows a schematic configuration of an artificial knee joint and a prosthetic limb according to an embodiment of the present invention;
Fig. 2 shows a schematic configuration of an artificial knee joint and a prosthetic limb according to an embodiment of the present invention;
Fig. 3 schematically shows transition of the knee angle θ, the thigh angle Ψ, and the shin angle Φ in accordance with the phase of walking of the wearer of the prosthetic limb;
Fig. 4 shows the cylinder and the control mechanism;
Figs. 5A and 5B show the flow of the oil in the bending motion and the stretching motion of the artificial knee joint;
Fig. 6 schematically shows functional blocks that provide bend control of the artificial knee joint;
Fig. 7 shows load lines L in the phases of walking of a wearer of the prosthetic limb; and
Fig. 8 is a functional block diagram of an adjustment support apparatus for the artificial knee joint.

The invention will now be described by reference to the preferred embodiments. This does not intend to limit the scope of the present invention, but to exemplify the invention.

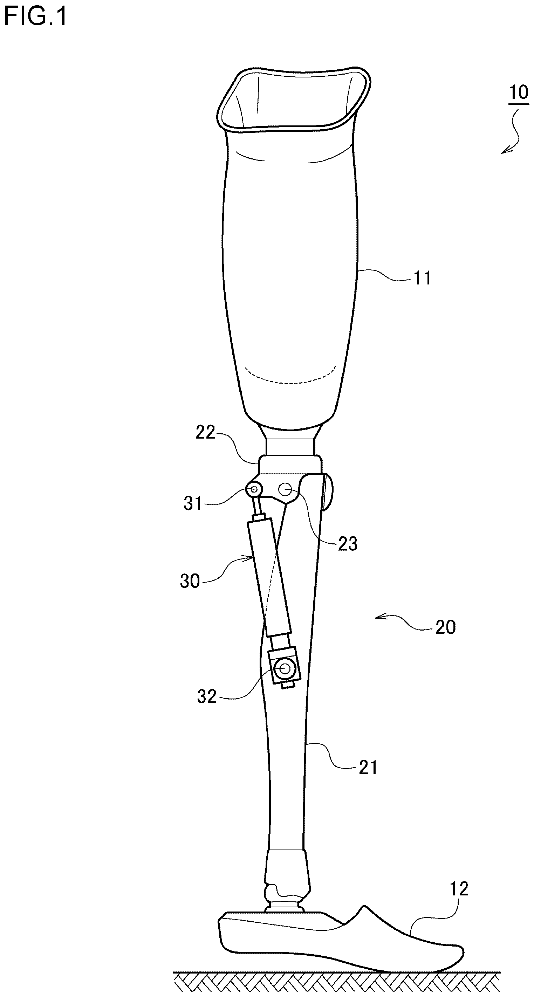

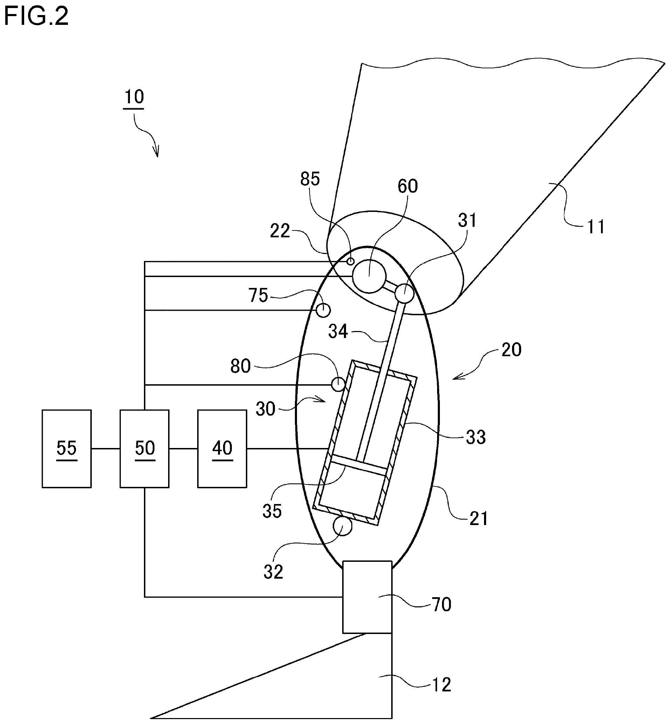

Figs. 1 and 2 show a schematic configuration of an artificial knee joint 20 and a prosthetic limb 10 according to an embodiment of the present invention. The prosthetic limb 10 is provided with a plastic socket 11 as a thigh part, a thigh joint part 22 to which the socket 11 is connected, a lower leg part 21 coupled to the thigh joint part 22 and provided to be rotatable around a knee shaft 23, and a foot part 12 coupled to the lower end of the lower leg part 21. The thigh joint part 22 to which the socket 11 is connected and the lower left part 21 bend or stretch the artificial knee joint 20 corresponding to the knee joint by relatively rotating around the knee shaft 23 provided in the joint between the thigh joint part 22 and the lower leg part 21 and perpendicular to the plane of Fig. 1. Further, the foot part 12 is formed by an elastic member, and the relative posture under a small load from the ground (e.g., when the foot part 12 does not touch the ground or when the wearer stands upright) is maintained constant by elasticity. Further, the elastic member is elastically deformed under a heavy load from the ground (e.g., when the wearer is walking) to produce a propelling power to kick the ground.

The artificial knee joint 20 is provided with the lower leg part 21 formed by a high-strength frame, the thigh joint part 22 connected to the socket 11 as a thigh part and coupled to the lower leg part 21 so as to be rotatable around the knee shaft 23, a cylinder 30 that restricts or permits the rotational motion around the knee shaft 23, i.e., the bending and stretching motion of the artificial knee joint 20, and a control mechanism 40 for driving the cylinder 30. Fig. 1 shows the artificial knee joint 20 in which the thigh joint part 22 and the lower leg part 21 are joined by a single link and which is rotatable around the single knee shaft 23 at a particular position. The present invention is equally applicable to the artificial knee joint 20 in which the thigh joint part 22 and the lower leg part 21 are joined by, for example, two front and rear links, and which is rotatable around the knee shaft 23 as a rotational center virtually and instantaneously formed inward of the total of four joints.

The amount of extension/contraction of the cylinder 30 and the knee angle, which is a rotational angle around the knee shaft 23 are substantially in one-to-one correspondence. A knee angle sensor 60 as a knee angle acquisition unit for measuring the amount of extension/contraction of the cylinder 30 to detect the knee angle of the artificial knee joint 20 is provided in the vicinity of the cylinder 30 and the thigh joint part 22. The knee angle detected by the knee angle sensor 60 is used by a control unit 50. The knee angle sensor 60 may be formed by an arbitrary sensor capable of measuring the amount of extension/contraction of the cylinder 30. For example, the knee angle sensor 60 may be formed by a Hall device capable of sensing the position of a magnet embedded in a piston rod 34 that moves in association with the extension/contraction of the cylinder 30. The knee angle is an angle formed by the axis of the socket 11 as the thigh part and the axis of the lower leg part 21. As shown in Fig. 1, for example, the knee angle formed when the user of the prosthetic limb 10 is standing upright and the axis of the socket 11 and the axis of the lower leg part 21 are aligned is 0 degrees. Further, the knee angle, formed when the user of the prosthetic limb 10 is seated, the axis of the lower leg part 21 remains aligned in the vertical direction of Fig. 1, and the axis of the socket 11 changes to the horizontal direction, is 90 degrees.

An inertial sensor 75 provided in the lower leg part 21 senses the posture and motion of the lower leg part 21 by measuring the speed (angular speed) and/or acceleration (angular acceleration) in the translational direction and/or rotational direction of the three axes defining the motion of the lower leg part 21. As described later, the shin angle, which defines the posture of the lower leg part 21 sensed by the inertial sensor 75 and which is a inclination angle formed by the axis of the lower leg part 21 relative to the vertical line passing through the knee shaft 23, is used to control the artificial knee joint 20 according to the embodiment. The shin angle and the thigh angle, which is described later, can be sensed in two directions including the longitudinal direction and the transversal direction and can be used to control the artificial knee joint 20. Hereinafter, the terms shin angle and thigh angle shall refer to an inclination angle in the longitudinal direction unless otherwise specified. The inertial sensor 75 capable of sensing the shin angle forms a lower leg part inclination angle acquisition unit of the present invention. The inertial sensor 75 can be provide at an arbitrary position of the lower leg part 21. For example, the inertial sensor 75 is implemented on a control board provided in the outer circumference of the cylinder 30 along with the control unit 50.

The thigh angle, which is an inclination angle formed by the axis of the socket 11 as the thigh part relative to the vertical line passing through the knee shaft 23, can be computed from the knee angle acquired by the knee angle sensor 60 and the shin angle acquired by the inertial sensor 75. In other words, the shin angle is the inclination of the lower leg part 21 from the vertical line passing through the knee shaft 23, and the knee angle is the inclination of the axis of the socket 11 from the axis of the lower leg part 21. By totaling the two, therefore, the thigh angle, which is the inclination of the axis of the socket 11 from the vertical line passing through the knee shaft 23, can be computed. Therefore, the knee angle sensor 60 and the inertial sensor 75 form a thigh part inclination angle acquisition unit of the present invention for acquiring the thigh angle. Alternatively, an inertial sensor may be provided in the socket 11 or the thigh joint part 22 to measure the thigh angle directly. In this case, the shin angle can be computed based on the knee angle and the thigh angle measured. Similarly, even if the knee angle sensor 60 is not provided, the knee angle can be computed by measuring the thigh angle and the shin angle.

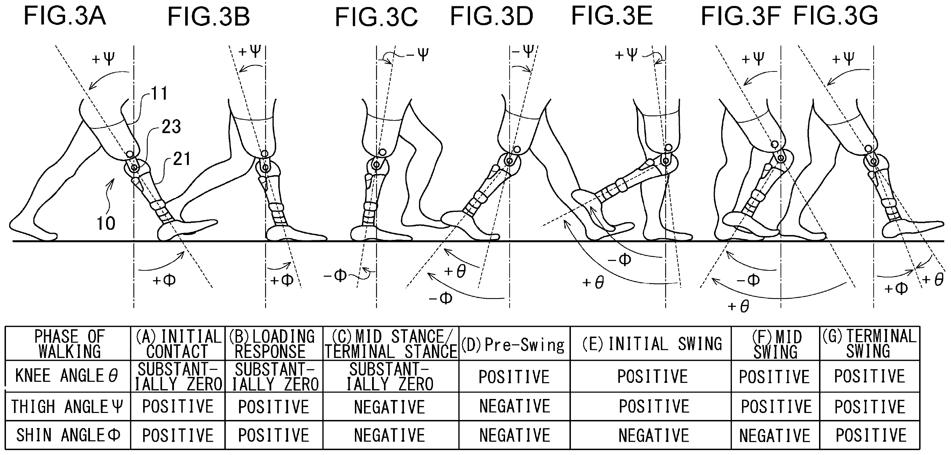

Fig. 3 schematically shows a transition of the knee angle θ, the thigh angle Ψ, and the shin angle Φ in accordance with the phase of walking (A)-(B) of the wearer of the prosthetic limb 10. The phase of walking (A) is the Initial Contact (IC) phase in which the prosthetic limb touches the ground. The phase of walking (B) is the Loading Response (LR) phase in which the weight of the wearer is supported by the prosthetic limb 10 touching the ground. The phase of walking (C) is the Mid Stance (MSt) to Terminal Stance (Tst) phase during which the gravitational center of the wearer moves ahead of the prosthetic limb 10 while the prosthetic limb 10 is supporting the weight. The phase of walking (D) is the Pre-Swing (PSw) phase in which the prosthetic limb 10 starts kicking the ground to make a transition to the subsequent Swing phase. The phases of walking (E), (F), and (G) are the Initial Swing (ISw), Mid Swing (MSw), and Terminal Swing (TSw) phases, respectively, in which the prosthetic limb 10 having left the ground is swung from back to front.

The thigh angle Ψ is defined to be negative (illustrated as -Ψ) when the socket 11 is inclined forward around the knee shaft 23 with reference to the vertical line passing through the knee shaft 23 and is defined to be positive (illustrated as +Ψ) when the socket 11 is inclined rearward. The shin angle Φ is defined to be positive (illustrated as +Φ) when the lower leg part 21 is inclined forward around the knee shaft 23 with reference to the vertical line passing through the knee shaft 23 and is defined to be negative (illustrated as -Φ) when the lower leg part 21 is inclined rearward. The knee angle θ is defined to be positive (illustrated as +θ) when the lower leg part 21 is inclined rearward around the knee shaft with reference to the axis of the socket 11. The knee angle θ, the thigh angle Ψ, and the shin angle Φ defined as described above always meet the relational expression "θ+Φ=Ψ".

As illustrated, the knee angle θ is substantially zero/the thigh angle Ψ is positive/the shin angle Φ is positive in the Initial Contact phase (A), the knee angle θ is substantially zero/the thigh angle Ψ is positive/the shin angle Φ is positive in the Loading Response phase (B), the knee angle θ is substantially zero/the thigh angle Ψ is negative/the shin angle Φ is negative in the Mid Stance/Terminal Stance phase (C), the knee angle θ is positive/the thigh angle Ψ is negative/the shin angle Φ is negative in the Pre-Swing phase (D), the knee angle θ is positive/the thigh angle Ψ is positive/the shin angle Φ is negative in the Initial Swing phase (E), the knee angle θ is positive/the thigh angle Ψ is positive/the shin angle Φ is negative in the Mid Swing phase (F), and the knee angle θ is positive/the thigh angle Ψ is positive/the shin angle Φ is positive in the Terminal Swing phase (G).

To focus on the knee angle θ, the knee angle θ increases and decreases monotonically such that the knee angle θ is substantially zero in the Stance phases (A)-(C) in which the prosthetic limb 10 in contact the ground supports the weight of the wearer, the knee angle θ maintains a value from substantially zero to positive in the Pre-Swing phase (D) between the Stance phases (A)-(C) and the Swing phases (E)-(G), and has a maximum value in the Mid-Swing phase (F) of the Swing phases (E)-(G), in which the prosthetic limb 10 leaves the ground and swung.

Control of the knee angle θ like this, i.e., bend control of the artificial knee joint 20, is performed by the control unit 50 via the control mechanism 40 and the cylinder 30. In other words, the knee angle θ is maintained to be substantially zero in the Stance phases (A)-(C) by increasing the hydraulic resistance of the cylinder 30 and restricting the rotation around the knee shaft 23 to prevent the artificial knee joint 20 from being bent by the weight of the wearer. In the Pre-Swing phase (D), the hydraulic resistance of the cylinder 30 is reduced to permit the rotation around the knee shaft 23 so as to make it possible for the artificial knee joint 20 to start bending in preparation for the subsequent Swing phases (E)-(G), which ensures that the knee angle θ has a positive value. In the Swing phases (E)-(G), the hydraulic resistance of the cylinder 30 is maintained to be low to allow the artificial knee joint 20 to be bent easily so that the prosthetic limb 10 swung away from the ground does not contact the ground. Details of bend control of the artificial knee joint 20 will be described later.

A description of the prosthetic limb 10 will be continued by referring back to Fig. 1 and Fig. 2. A load sensor 70 for detecting the load exerted between the lower leg part 21 and the foot part 12 may be provided at the lower end of the lower leg part 21, as a sensor other than the knee angle sensor 60 and the inertial sensor 75. Since it is possible to recognize the phase of walking of the wearer of the prosthetic limb 10 based on the magnitude and direction of the load detected by the load sensor 70, the load sensor 70 may be used for bend control of the artificial knee joint 20 in addition to the knee angle sensor 60 and the inertial sensor 75. The load sensor may be provided in the thigh joint part 22 or in the knee part between the thigh joint part 22 and the lower leg part 21. Meanwhile, as described later, bend control of the artificial knee joint 20 can be performed by using the knee angle sensor 60 and the inertial sensor 75 only so that the load sensor 70 may be omitted to configure the artificial knee joint 20 inexpensively. Further, a temperature sensor 80 for measuring the temperature of the oil in the cylinder 30 is attached to the outer wall or the inner wall of the cylinder 30. The measurement information of these sensors is used in the control unit 50.

A vibrator 85 is provided in the thigh joint part 22 or the lower leg part 21. The vibrator 85 notifies or alerts the user wearing the prosthetic limb 10 by vibration and is controlled by the control unit 50.

The control unit 50 controls the control mechanism 40 based on the measurement information of various sensor such as the knee angle sensor 60, the load sensor 70, the inertial sensor 75, and the temperature sensor 80 to control the resistance to the extension/contraction motion of the cylinder 30, i.e., the rotational resistance of the knee shaft 23 during the bending motion of the artificial knee joint 20. A battery 55 for supplying power to the parts of the artificial knee joint 20 is connected to the control unit 50. Fig. 2 shows the control mechanism 40, the control unit 50, and the battery 55 outside the artificial knee joint 20 but in practice are provided inside the lower leg part 21 as components constituting the artificial knee joint 20.

The cylinder 30 is a hydraulic cylinder that restricts or permits the bending or stretching motion of the artificial knee joint 20 by producing a drag by using oil as a working fluid. The cylinder 30 is supported by an upper support point 31 provided in the vicinity of the knee shaft 23 that rotatably couples the socket 11 and the lower leg part 21 and by a lower support point 32 coupled to a part of the lower leg part 21 and can extend or contract between the support points. In the compression step in which the cylinder length is reduced, a bending motion in which the artificial knee joint 20 is rotated counterclockwise of Fig. 1 around the knee shaft 23 is performed. In the expansion step in which the cylinder length is enlarged, a stretching motion in which the artificial knee joint 20 is rotated clockwise of Fig. 1 around the knee shaft 23 is performed. The cylinder length refers to a length between the upper support point 31 and the lower support point 32 of the cylinder 30.

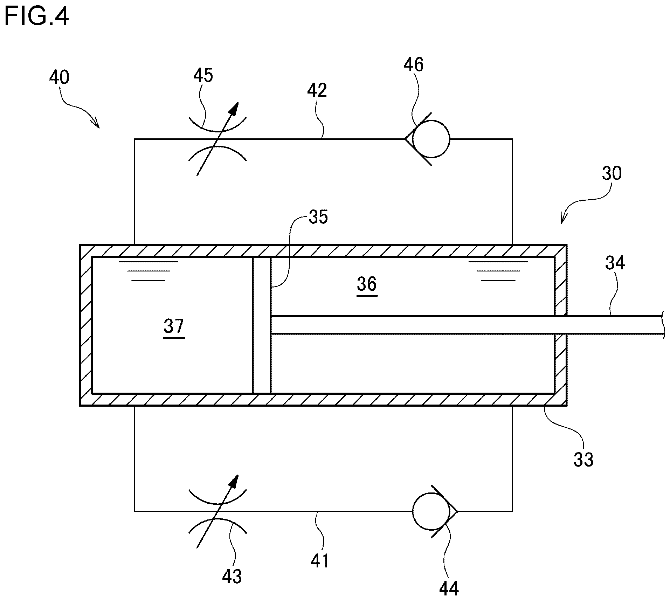

A description will now be given of the cylinder 30 and the control mechanism 40 with reference to Fig. 4. The cylinder 30 is provided with a cylinder tube 33, a piston rod 34 inserted from the side of one end (the right end side of Fig. 4) of the cylinder tube 33 and movable along the longitudinal direction (the transversal direction of Fig. 4) of the cylinder tube 33, and a piston 35 secured to the piston rod 34 in the cylinder tube 33 and slidable in the longitudinal direction along the inner wall of the cylinder tube 33. The piston 35 partitions the interior of the cylinder tube 33 into a first cavity 36 on the side of the one end (the right end side of Fig. 4) and a second cavity 37 on the side of the other end (the left end side of Fig. 4). The first cavity 36 and the second cavity 37 are filled with oil as a working fluid.

The control mechanism 40 is a hydraulic driving mechanism for driving the cylinder into expansion or compression by a hydraulic pressure. The control mechanism 40 is provided with an stretching side hydraulic circuit 41 and a bending side hydraulic circuit 32 respectively connected to the cylinder 30. The stretching side hydraulic circuit 41 and the bending side hydraulic circuit 42 respectively communicate with the first cavity at one and communicate with the second cavity 37 at the other end. The stretching side hydraulic circuit 41 is provided with a stretching side valve 42 as a valve capable of opening or closing the fluid path of the oil for producing the rotational resistance of the knee shaft 23 and with a stretching side check valve 44. Opening the stretching side valve 43 allows the oil to be distributed in the stretching side hydraulic circuit 41. The action of the stretching side check valve 44 causes the oil to flow only in the direction from the first cavity 36 to the second cavity 37 and not to flow in the opposite direction.

The bending side hydraulic circuit 42 is provided with a bending side valve 45 as a valve capable of opening or closing the fluid path of the oil for producing the rotational resistance of the knee shaft 23 and with a bending side check valve 46. Opening the bending side valve 45 allows the oil to be distributed in the bending side hydraulic circuit 42. The action of the bending side check valve 46 causes the oil to flow only in the direction from the second cavity 37 to the first cavity 36 and not to flow in the opposite direction. The positions of the stretching side valve 43 and the bending side valve 45 are individually controlled by the control unit 50. The position of each valve can have an arbitrary value between fully open (the most open position) and fully closed (the least open position). When the valve is fully closed, the flow of the oil is interrupted so that the hydraulic pressure is maximized. The more open the valve toward full open, the larger the cross section through which the oil can be distributed in the valve and the smaller the hydraulic resistance.

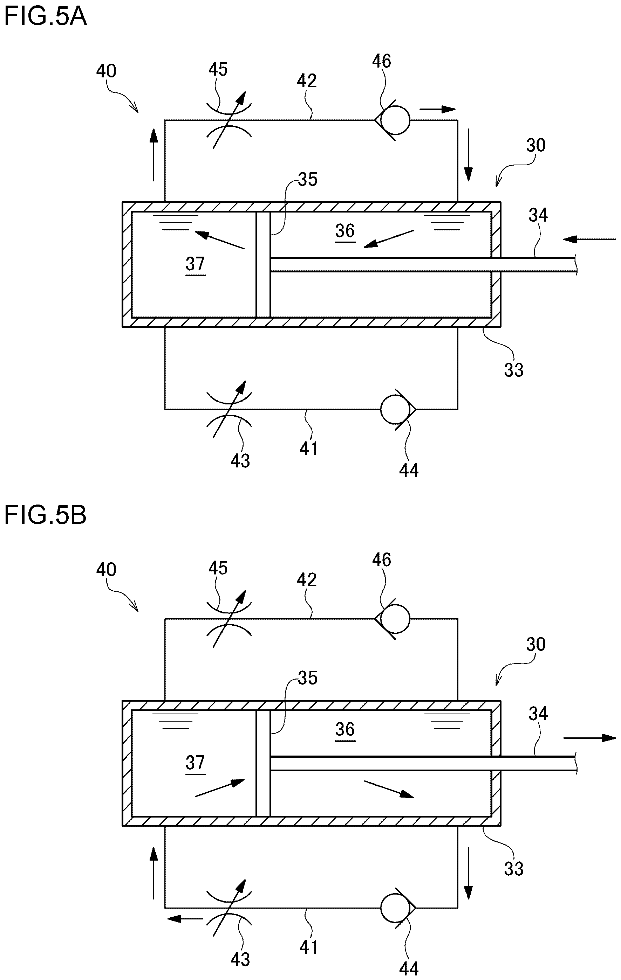

Fig. 5A shows the flow of the oil in the bending motion of the artificial knee joint 20. Bending is a compression step in which the cylinder length is decreased. The piston rod 34 recedes leftward in Fig. 5, and the piston 35 moves toward the intake side. The oil extruded from the second cavity 37 by the movement of piston 35 cannot be distributed in the stretching side hydraulic circuit 41 provided with the stretching side check valve 44 and so flows into the first cavity 36 via the bending side hydraulic circuit 42. Controlling the bending side valve 45 to be less open in this process makes it difficult for the oil to flow in the bending side hydraulic circuit 42 and so can restrict the bending motion of the artificial knee joint 20. Thus, the control unit 50 constitutes the rotational resistance control unit of the present invention for controlling the rotational resistance of the knee shaft 23 during the bending motion of the artificial knee joint 20, by controlling the position of the bending side valve 45.

Fig. 5B shows the flow of the oil in the stretching motion of the artificial knee joint 20. Stretching is an expansion step in which the cylinder length is increased. The piston rod 34 advances rightward in Fig. 5, and the piston 35 moves toward the extrusion side. The oil extruded from the first cavity 36 by the movement of piston 35 cannot be distributed in the bending side hydraulic circuit 42 provided with the bending side check valve 46 and so flows into the second cavity 37 via the stretching side hydraulic circuit 41. Controlling the stretching side valve 43 to be less open in this process makes it difficult for the oil to flow in the stretching side hydraulic circuit 41 and so can restrict the stretching motion of the artificial knee joint 20. Thus, the control unit 50 constitutes the rotational resistance control unit of the present invention for controlling the rotational resistance of the knee shaft 23 during the stretching motion of the artificial knee joint 20, by controlling the position of the stretching side valve 43.

Reference is made back to Fig. 2, and the sensors provided in the prosthetic limb 10 will be described further.

The knee angle sensor 60 measures the position of the piston rod 34 extended or contracted. For example, the position of a magnet attached to the piston rod 34 is measured by a magnetic sensor provided in the cylinder tube 33. The position of the piston rod 34 extended or contracted and the knee angle of the artificial knee joint 20 or the rotational angle of the knee shaft 23 are in one-to-one correspondence. Therefore, the knee angle sensor 60 can convert the detected position of the piston rod 34 extended or contracted into the knee angle of the artificial knee joint 20. The computation to convert the position of the piston rod 34 extended or contracted into the knee angle of the artificial knee joint 20 may be performed in the control unit 50. The knee angle sensor 60 in this case measures the position of the piston rod 34 extended or contracted and provides the measurement to the control unit 50.

The inertial sensor 75 senses the posture and motion of the lower leg part 21 based on the measured speed and/or acceleration of the respective axes. For example, the phase of walking of the wearer of the prosthetic limb 10 can be recognized from the variation in the speed/acceleration measured by the inertial sensor 75, and the position of the lower leg part 21 during waling can be tracked by integrating measurement values of the inertial sensor 75. Therefore, the step length, which is an amount of displacement per step, and the walking speed per step can be determined. The computation to determine the position by integrating measurement values of the inertial sensor 75, etc. is performed by the control unit 50.

The load sensor 70 is comprised of, for example, a strain sensor and is provided in the ankle part between the lower leg part 21 and the foot part 12. The load exerted on the ankle part produces a strain in the object that constitutes the strain sensor so that the load can be measured by detecting the strain. By providing a load sensor like this in the thigh joint part 22, the load exerted on the knee joint can also be measured. The computation to convert the strain detected by the strain into the load may be performed by the control unit 50. The load sensor 70 in this case provides the detected strain to the control unit 50. Further, the control unit 50 can recognize the phase of walking of the wearer of the prosthetic limb 10 from the variation in the magnitude and direction of the load measured by the load sensor 70 and so can determine the step length and the walking speed by computation. Also, information on the moment exerted on the artificial knee joint 20 can be obtained from the position, magnitude, direction of the load measured by the load sensor 70.

The temperature sensor 80 measures the temperature of the cylinder 30 or the temperature of the oil in the cylinder 30. For example, the temperature sensor 80 initiates a transition to a high-temperature mode in which the action of the artificial knee joint 20 is restricted upon detecting that the cylinder 30 reaches a high temperature due to the head from the hydraulic resistance. Further, depending on the physical property of the oil, the hydraulic resistance varies in accordance with the temperature variation. Therefore, the control unit 50 can realize a desired hydraulic resistance by controlling the control mechanism 40 in accordance with the temperature measured by the temperature sensor 80. More specifically, control data set for the control mechanism 40 to realize the respective values of hydraulic resistance is created for each temperature and stored in the control unit 50. The control unit 50 selects the control data set corresponding to the temperature measured by the temperature sensor 80 and uses the data for control.

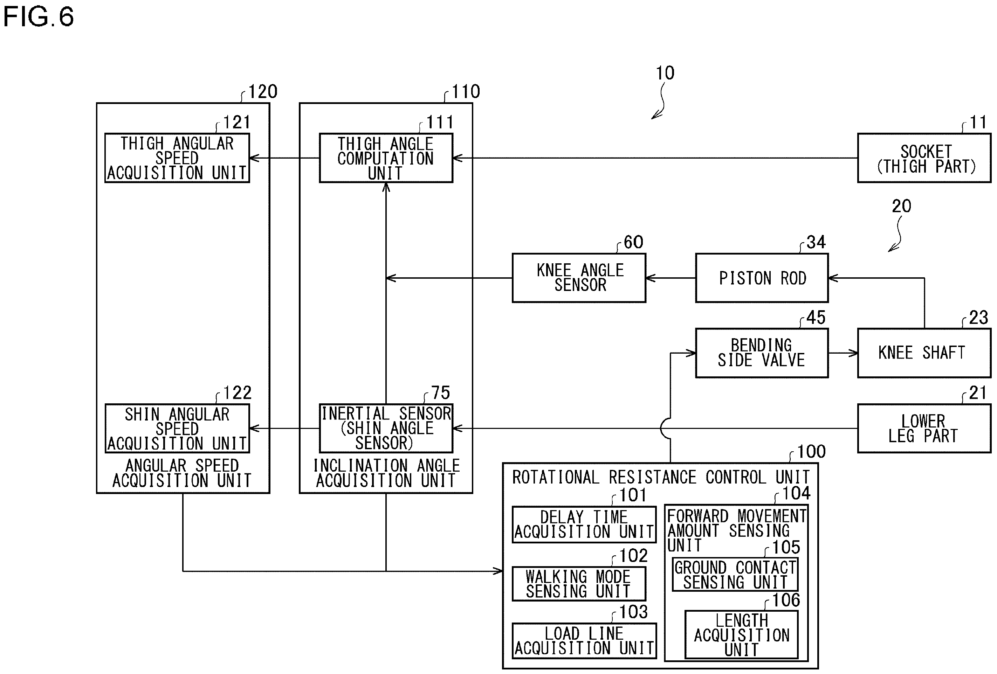

Fig. 6 schematically shows functional blocks that provide bend control of the artificial knee joint 20. The figure shows only those constituting elements of the prosthetic limb 10 related to bend control of the artificial knee joint 20. Further, the illustrated constituting elements are provided in the artificial knee joint 20 except for the socket 11. The constituting elements that provide information processing such as a rotational resistance control unit 100 are implemented in the control unit 50.

An inclination angle acquisition unit 110 is provided with a thigh angle computation unit 111 that acquires the thigh angle Ψ, which is an inclination angle formed by the socket 11 as the thigh part relative to the vertical line passing through the knee shaft 23, constantly or at a predetermined time interval and with the inertial sensor 75 as a shin angle sensor that acquires the shin angle Φ, which is an inclination angle formed by the lower leg part 21 relative to the vertical line passing through the knee shaft 23, constantly or at a predetermined time interval. As described above, the knee angle θ, the thigh angle Ψ, and the shin angle Φ meet the relational expression "θ+Φ=Ψ" so that the thigh angle computation unit 111 can compute the thigh angle Ψ=θ+Φ based on the knee angle θ measured by the knee angle sensor 60 and the shin angle Φ measured by the inertial sensor 75.

An angular speed acquisition unit 120 is provided with a thigh angular speed acquisition unit 121 (a thigh part inclination angular speed acquisition unit) that acquires the angular speed of the thigh angle Ψ and a shin angular speed acquisition unit 122 that acquires the angular speed of the shin angle Φ. More specifically, the thigh angular speed acquisition unit 121 determines the thigh angular speed by differentiating the thigh angle Ψ computed by the thigh angle computation unit 111 with respect to time, and the shin angular speed acquisition unit 122 determines the shin angular speed by differentiating the shin angle Φ measured by the inertial sensor 75 with respect to time. When the inertial sensor 75 as a shin angle sensor can measure the shin angular speed directly, the inertial sensor 75 itself constitutes the shin angular speed acquisition unit 122. Alternatively, the thigh angular speed may be computed based on the shin angular speed acquired by the shin angular speed acquisition unit 122 and the knee angular speed determined by differentiating the knee angle θ measured by the knee angle sensor 60 with respect to time.

The rotational resistance control unit 100 controls the rotational resistance (hereinafter, bend resistance) of the knee shaft 23 during the bending motion of the artificial knee joint 20, by controlling the position of the bending side valve 45. The rotational resistance control unit 100 is provided with, as constituting elements for acquiring various information used for control of bend resistance, a delay time acquisition unit 101, a walking mode sensing unit 102, a load line acquisition unit 103, and a forward movement amount sensing unit 104. The rotational resistance control unit 100 need not be provided with all of these functional units but may be provided with at least one of the function units.

The delay time acquisition unit 101 acquires a delay time that elapses until the result of control of the bending side valve 45 by the rotational resistance control unit 100 is reflected in the actual bend resistance. The delay time is primarily determined by the control period of the rotational resistance control unit 100 and by the driving period during which the motor is driven to open or close the bending side valve 45 to a desired position. Given, for example, that the control period of the rotational resistance control unit 100 is 50 ms and the driving period of the motor is 50 ms, a delay time of about 100 ms in total is produced. As described later, the bend resistance is controlled in the embodiment by allowing for the delay time as well.

The walking mode sensing unit 102 senses the walking mode of the user wearing the prosthetic limb 10 based on the posture and motion of the respective parts of the prosthetic limb 10 that can be sensed by the knee angle sensor 60, the inertial sensor 75, the thigh angle computation unit 111, the angular speed acquisition unit 120, etc. For example, the walking mode sensing unit 102 can sense that the wearer of the prosthetic limb 10 is walking forward ordinarily as shown in Fig. 3 and can sense the phases of walking (A)-(G) during the walk. The walking mode acquisition unit 102 can also sense a particular walking mode different from the ordinary walking mode of Fig. 3. For example, the walking mode acquisition unit 102 can sense an abnormal walk mode such as backward walking and a forward, backward, leftward, rightward, or upward jump.

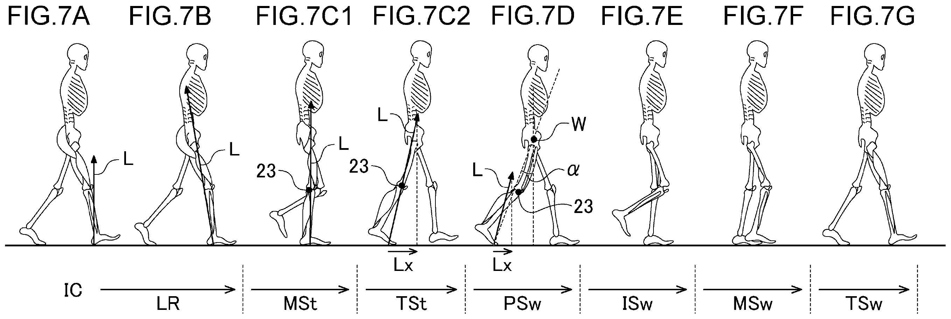

The load line acquisition unit 103 acquires a load line L indicating the direction of load exerted on the lower leg part 21. Fig. 7 shows load lines L in the phases of walking, equally shown in Fig. 3, as arrows or vectors. The length of the load line L represents the magnitude of load. The phases of walking (A)-(G) of Fig. 7 correspond to the phases of walking (A)-(G) of Fig. 3, but the Mid Stance/Terminal Stance phase shown as one phase of walking (C) in Fig. 3 is divided into two phase of walking, i.e., the Mid Stance MSt phase (C1) and the Terminal Stance Tst phase (C2), in Fig. 7.

To focus on the relationship between the load line L and the knee shaft 23 in the phases of walking (C1)-(D), the knee shaft 23 (the virtual and instantaneous rotational center in the artificial knee joint 20 in which the thigh joint part 22 and the lower leg part 21 are joined by a plurality of links) is slightly behind the load line L in the Mid Stance phase (C1). The knee shaft 23 is on the load line L in the Terminal Stance phase (C2), and the knee shaft 23 is in front of the load line L in the Pre-Swing phase (D). Thus, the knee shaft 23 makes a transition from a point behind the load line L to a point in front of the load line L, in association with the transition between the phases of walking (C1)-(D). Like the walking mode described above, the load line L is determined based on the posture and motion of the respective parts of the prosthetic limb 10 that can be sensed by the inertial sensor 75, the thigh angle computation unit 111, the angular speed acquisition unit 120, etc. Further, the load line L can be measured directly by the sensor 70 when the load sensor 70 is provided in the artificial knee joint 20.

The forward movement amount sensing unit 104 senses the amount of forward movement of the user wearing the artificial knee joint 20. As shown in the Terminal Stance phase (C2) and the Pre-Swing phase (D) of Fig. 7, the component Lx of the load line in the horizontal direction or the direction of travel is the forward movement amount, which represents an advancing force from the load that contributes to the advancement of the wearer of the prosthetic limb 10. It is sufficient if the variation or increase/decrease of the forward movement amount is known for control of bend resistance by the rotational resistance control unit 100. It is not necessary to sense the forward movement amount or the advancing force strictly. Therefore, it is sufficient for the forward movement amount sensing unit 104 to acquire data correlated to the forward movement amount.

Data indicative of the forward movement amount is exemplified by the thigh angular speed acquired by the thigh angular speed acquisition unit 121. In association with the occurrence of a forward movement amount in the Terminal Stance phase (C2) and the Pre-Swing phase (D) of Fig. 7, the thigh angular speed has a negative value in the Mid Stance/Terminal Stance phase (C) and the Pre-Swing phase (D) of Fig. 3. Therefore, a negative thigh angle speed suggests a forward movement amount.

Data indicative of the forward movement amount can also be obtained from a ground contact sensing unit 105 or a length acquisition unit 106. The ground contact sensing unit 105 senses the contact of the artificial knee joint 20 with the ground. The length acquisition unit 106 acquires the length of the artificial knee joint 20 from the thigh joint part 22 to the lower end of the lower leg part 21. In the Terminal Stance phase (C2) and the Pre-Swing phase (D) in which a forward movement amount occurs, the leg wearing the artificial knee joint 20 is in contact with the ground, and the line connecting the toe and the waist W is inclined forward. It can therefore be said that a forward movement amount is present when the angle α of forward inclination of the waist W has increased relative to the vertical line when the ground contact sensing unit 105 senses that the artificial knee joint 20 is in contact with the ground.

When the artificial knee joint 20 is provided with the load sensor 70 as a load sensing unit, the ground contact sensing unit 105 can directly sense that the artificial knee joint 20 is in contact with the ground by referring to the load measured by the load sensor 70. However, it might not be possible to distinguish between an ordinary walk and a backward walk merely by the load vertically above sensed by the load sensor 70. In this case, the two modes of walking can be distinguished according to the thigh angle Ψ computed by using the inertial sensor 75. Specifically, in an ordinary walk as shown in Fig. 3, the thigh angle Ψ maintains a large positive value in the phases of walking (G) through (A), in which the prosthetic limb 10 is in contact with the ground, but, in a backward walk, the thigh angle Ψ makes a transition from a positive value to a negative value in the phases of walking (E) through (D), in which the prosthetic limb 10 is in contact with the ground. Therefore, the two modes of walking can be distinguished. It is ensured that control to weaken the rotational resistance of the knee shaft 23 is not performed when a backward walk is detected.

When the artificial knee joint 20 is not provided with the load sensor 70, on the other hand, the ground contact sensing circuit 105 can sense whether the foot part 12 is in contact with the ground according to the posture of the respective parts of the prosthetic limb 10 (the foot part 12, the lower leg part 21, the knee shaft 23, the thigh joint part 22, the socket 11, etc.) that can be sensed by referring to the measurement data of the inertial sensor 75 and the knee angle sensor 60 that function as a load sensing unit and according to geometrical computation based on the length of the artificial knee joint 20 acquired by the length acquisition unit 106. Further, the angle α of forward inclination α of the waist W can be approximately computed from the shin angle Φ, the knee angle θ, the thigh angle Ψ, the length of the artificial knee joint 20 acquired by the length acquisition unit 106, etc. When the inertial sensor 75, which has not detected a load, detects an abrupt acceleration vertical above, it can be assumed that the foot part 12 moving downward hits the ground and comes to a stop, which permits a determination that the artificial knee joint 20 was not in contact with the ground previously. In this case, it is determined that the user walks backward when the thigh angle Ψ makes a transition from a positive value to a negative value, and control of the rotational resistance of the knee shaft 23 to be weaker is prohibited. Alternatively, the rotational resistance of the knee shaft 23 is controlled to be stronger if it has already been weakened.

A description will now be given of specific bend control of the artificial knee joint 20 by the rotational resistance control unit 100. As described above with reference to Fig. 3, the rotational resistance control unit 100 maintains the knee angle θ to be substantially zero in the Stance phases (A)-(C), by controlling the bending side valve 45 to be less open to strength the bend resistance and restricting the rotation around the knee shaft 23, in order to prevent the artificial knee joint 20 from being bent under the weight of the wearer. In the Pre-Swing phase (D), the rotational resistance control unit 100 controls the bending side valve 45 to be more open to weaken the bend resistance gradually and permit the rotation around the knee shaft 23 so as to make it possible for the artificial knee joint 20 to start bending in preparation for the subsequent Swing phases (E)-(G), which ensures that the knee angle θ has a positive value. In the Swing phases (E)-(G), the rotational resistance control unit 100 maintains the bending side valve 45 to be highly open and maintains a state in which the bend resistance is weak and the artificial knee joint 20 is easily bent so as to prevent the prosthetic limb 10 swung away from the ground does not contact the ground.

For the purpose of realizing a smooth walk of the wearer of the prosthetic limb 10, it is particularly important to cause the bending side valve 45 to make a transition from a less open state to a more open state and to cause the bend resistance to make a transition from a strong state to a weak state in the Mid Stance/Terminal Stance phase (C) or the Pre-Swing phase (D). If the bend resistance is weakened too early, an irregular action of the wearer of the prosthetic limb 10 such as an abrupt stop increases the likelihood of "knee instability" in which the artificial knee joint 20 that has made a transition to a state of weak bend resistance is bent unintentionally. If the bend resistance is weakened too late, on the other hand, the bend resistance remains strong even in the Initial Swing phase (E) to prohibit the artificial knee joint 20 from being bent so that the likelihood that the prosthetic limb 10 swung away in an stretched state contacts the ground is increased.

The embodiment addresses this by determining the timing of weakening the bend resistance in the Mid Stance/Terminal Stance phase (C) or the Pre-Swing phase (D) according to various criteria listed below. Each of the criteria contributes to resolution of the above issue alone, but the timing of weakening the bend resistance can be optimized by combining these criteria as appropriate.

According to the first criterion, the rotational resistance control unit 100 weakens the bend resistance of the knee shaft 23 in accordance with a transition of the thigh angle Ψ computed by the thigh angle computation unit 111 from positive to negative. As shown in Fig. 3, the thigh angle Ψ makes a transition from positive to negative from the Loading Resistance phase (B) to the Mid Stance/Terminal Stance phase (C). Therefore, the artificial knee joint 20 in which the bend resistance is weakened in association with this can make a smooth transition to the Swing phases (D)-(G). The point of time when the rotational resistance control unit 100 starts controlling the bend resistance of the knee shaft 23 to be weaker, i.e., the point of time when the rotational resistance control unit 100 starts controlling the bending side valve 45 to be more open, may be a point of time when the thigh angle Ψ decreasing from positive to negative reaches 0 degrees, a point of time when the thigh angle Ψ falls below a negative threshold value, a point of time when the thigh angle Ψ starts to decrease, after a predetermined period of time elapses since the thigh angle Ψ starts to decrease, a point of time when the amount of decrease since the thigh angle Ψ starts to decrease is equal to greater than a predetermined amount, or a point of time when the decrease rate acquired by the thigh angular speed acquisition unit 121 is equal to or greater than a predetermined value.

The rotational resistance control unit 100 determines a point of time to start controlling the bend resistance of the knee shaft 23 to be weaker by also allowing for the delay time acquired by the delay time acquisition unit 101. In other words, when the angular speed of the thigh angle Ψ acquired by the thigh angular speed acquisition unit 121 is negative, the rotational resistance control unit 100 starts controlling the rotational resistance of the knee shaft 23 to be weaker while the thigh angle Ψ is larger than a threshold value to prevent the thigh angle Ψ from falling below the threshold value during the delay time acquired by the delay time acquisition unit 101. As described above, given, for example, the control period of the rotational resistance control unit 100 is 50 ms and the driving period of the motor for opening or closing the bending side valve 45 is 50 ms, a delay time of 100 ms is produced at a maximum. Given that the thigh angle Ψ is +5 degrees and the thigh angular speed is -0.05 degrees/ms, the likelihood that the thigh angle Ψ becomes 0 degrees or negative during the delay time of 100 ms (0.05 degrees/ms×100 ms=5 degrees) is high. Thus, even when the thigh angle Ψ is positive (+5 degrees), the rotational resistance control unit 100 starts controlling the bend resistance to be weaker in advance when recognizing the likelihood that the thigh angle Ψ becomes 0 degrees or negative during the delay time, by referring to the negative thigh angular speed (-0.05 degrees/ms) and the delay time (100ms).

According to the second criterion in isolation not part of the invention, the rotational resistance control unit 100 weakens the bend resistance of the knee shaft 23 before the knee shaft 23 makes a transition from a point behind the load line L acquired by the load line acquisition unit 103 to a point in front. As shown in Fig. 7, the knee shaft 23 makes a transition from a point behind of the load line L to a point in front of the load line L from the Mid Stance phase (C1) to the Pre-Swing phase (D). Therefore, the artificial knee joint 20 in which the bend resistance is weakened in association with this can make a smooth transition to the Pre-Swing phase (D) and the Swing phases (E)-(G). When the knee shaft 23 is behind the load line L (e.g., the Mid Stance phase (C1)), the knee shaft 23 is not bent regardless of the magnitude of the load. When the knee shaft 23 is in front of the load line L (e.g., the Pre-Swing phase (D)), on the other hand, the knee shaft 23 is bent provided that the load is greater than the bend resistance. Thus, the bend resistance is weakened, according to the second criterion, before the knee shaft 23 makes a transition from a point behind the load line L to a point in front so that the artificial knee joint 20 can be bent smoothly when the knee shaft 23 reaches a point in front of the load line L.

The point of time when the rotational resistance control unit 100 starts controlling the bend resistance of the knee shaft 23 to be weaker, i.e., the point of time when the rotational resistance control unit 100 starts controlling the bending side valve 45 to be more open, may be immediately before the knee shaft 23 moving from back to front reaches a point aligned to the load line L, a point of time when the knee shaft 23 starts a forward relative movement with respect to the load line L in the phases of walking (C1)-(D), after a predetermined period of time elapses since the knee shaft 23 starts a forward relative movement with respect to the load line L, a point of time when the amount of relative movement since the knee shaft 23 started a forward relative movement with respect to the load line L is equal to or greater than a predetermined amount, or a point of time when the forward relative speed of the knee shaft 23 with respect to the load line L is equal to or greater than a predetermined value. In consideration of the likelihood that the wearer of the prosthetic limb 10 loses balance suddenly, it is desired that control is started immediately before the knee shaft 23 reaches a point in front of the load line L. As in the case of the first criterion, the rotational resistance control unit 100 may determine a point of time to start controlling the bend resistance of the knee shaft 23 to be weaker by also allowing for the delay time acquired by the delay time acquisition unit 101.

According to the third criterion, the rotational resistance control unit 100 weakens the bend resistance of the knee shaft 23 when the first or second criterion is met, and when the angular speed of the thigh angle Ψ and/or the shin angle Φ acquired by the angular speed acquisition unit 120 remains negative for a predetermined period of time continuously. As shown in Fig. 3, the thigh angle Ψ and the shin angle Φ decrease and has a negative angular speed from the Loading Response phase (B) to the Pre-Swing phase (D9).

When the thigh angle Ψ and/or the shin angle Φ has a negative angular speed for a predetermined period of time continuously as described above, the likelihood of an ordinary forward walk as shown in Fig. 3 is high so that the rotational resistance control unit 100 is permitted to weaken the bend resistance of the knee shaft 23. Conversely, when the thigh angle Ψ or the shin angle Φ does not have a negative angular speed for a predetermined period of time continuously, it is likely that the wearer is walking in an abnormal manner (e.g., walking backward, jumping, etc.) different from the ordinary walking mode of Fig. 3, so that the wearer might fall due to knee instability if the bend resistance of the knee shaft 23 is weakened. Therefore, the rotational resistance control unit 100 secures the safety of the wearer of the prosthetic limb 10 by controlling the bending side valve 45 to be less open to strengthen the bend resistance.

According to the fourth criterion, the rotational resistance control unit 100 weakens the bend resistance of the knee shaft 23 when the first or second criterion is met and when the thigh angle Ψ and/or the shin angle Φ acquired by the inclination angle acquisition unit 110 at a predetermined time interval has decreased a predetermined number of times consecutively. The fourth criterion, which requires "the thigh angle Ψ and/or the shin angle Φ has decreased a predetermined number of times consecutively" is equivalent to the third criterion, which requires " the angular speed of the thigh angle Ψ and/or the shin angle Φ remains negative for a predetermined period of time continuously".

According to the fifth criterion in isolation not part of the invention, the rotational resistance control unit 100 weakens the bend resistance of the knee shaft 23 when the knee angle θ measured by the knee angle sensor 60 is equal to or smaller than a predetermined value. As shown in Fig. 3, the knee angle θ is substantially zero in the Mid Stance/Terminal Stance phase (C), in which the bend resistance should be weakened in an ordinary walk on a level ground. When the knee angle θ is equal to or smaller than a predetermined value at a point of time to weaken the bend resistance, the likelihood of an ordinary walk on a level ground is high so that the rotational resistance control unit 100 is permitted to weaken the bend resistance of the knee shaft 23.

Conversely, when the knee angle θ is larger than the predetermined value at a point of time to weaken the bend resistance, it is likely that the wearer is walking on a sloping road, etc., bending the artificial knee joint 20. If the bend resistance of the knee shaft 23 is weakened, the wearer might fall due to knee instability. Therefore, the rotational resistance control unit 100 secures the safety of the wearer of the prosthetic limb 10 by controlling the bending side valve 45 to be less open to strengthen the bend resistance. The fifth criterion may require, in addition to or in place of the requirement that the knee angle θ is equal or smaller than a predetermined value, that the angular speed of the knee angle θ is equal to or smaller than a predetermined value. During an ordinary walk on a level ground, the angular speed of the knee angle θ is substantially zero in the Stance phases (A)-(C). During a walk on a sloping road, etc. on the other hand, the knee angle θ varies in the Stance phases (A)-(C) and has an angular speed larger than the predetermined value. Therefore, the modes of walking can be distinguished effectively.

According to the sixth criterion in isolation not part of the invention, the rotational resistance control unit 100 does not control the bend resistance of the knee shaft 23 to be weaker even if any of the above criteria are met, provided that the walking mode sensed by the walking mode sensing unit 102 is a particular walking mode. More specifically, when an abnormal walking mode (e.g., backward walk, jump, etc.) different from the ordinary walking mode of Fig. 3 and Fig. 7 is sensed, the wearer might fall due to knee instability if the bend resistance of the knee shaft 23 is weakened. Therefore, the rotational resistance control unit 100 secures the safety of the wearer of the prosthetic limb 10 by controlling the bending side valve 45 to be less open to strengthen the bend resistance.

According to the seventh criterion in isolation not part of the invention, the rotational resistance control unit 100 strengthen the bend resistance of the knee shaft 23 even if any of the above criteria for weakening the bend resistance is met, provided that the forward movement amount sensed by the forward movement amount sensing unit 104 makes a transition from a state in which the forward movement amount sensed by the forward movement amount sensing unit 104 is equal to or larger than a predetermined forward movement amount threshold value to a state in which forward movement amount is smaller than the forward movement amount threshold value. When the wearer of the prosthetic limb 10 loses speed abruptly in the Terminal Stance phase (C2) of Fig. 7 so that the advancing force Lx is lost, for example, only the load in the vertical direction remains so that the load line L moves behind the knee shaft 23. If the bend resistance of the knee shaft 23 remains weakened in this state, the vertical load might cause instability of the artificial knee joint 20, which might lead to a fall of the wearer. Thus, the rotational resistance control unit 100 secures the safety of the wearer of the prosthetic limb 10 by controlling the bending side valve 45 to be less open to strengthen the bend resistance.

The forward movement amount threshold value can be set as desired. It is preferable that the forward movement amount threshold value be set based on data for the forward movement amount in a past round of walk of the user wearing the artificial knee joint 20. More specifically, the forward movement amount Lx or data indicative thereof that occurs in the Terminal Stance phase (C2) or the Pre-Swing phase (D) of Fig. 7 is measured in multiple rounds of walk under different conditions. The forward movement amount threshold value is set to an arbitrary value smaller than the measured amounts with significance. The bend resistance of the knee shaft 23 may be strengthened when the amount of decrease (loss) of the forward movement amount sensed by the forward movement amount sensing unit 104 exceeds a predetermined amount of decrease threshold value, or when the decrease rate (speed of loss) of the forward movement amount sensed by the forward movement amount sensing unit 104 exceeds a predetermined decrease rate threshold value.

According to the eighth criterion, control by the rotational resistance control unit 100 to weaken the bend resistance of the knee shaft 23 is enabled according to the above criteria, provided that the forward movement amount sensed by the forward movement amount sensing unit 104 is equal to or greater than a predetermined forward movement amount threshold value. In other words, when the forward movement amount Lx of a predetermined amount or greater is sensed, the likelihood of an ordinary forward walk as shown in Fig. 7 is high so that the rotational resistance control unit 100 is permitted to weaken the bend resistance of the knee shaft 23.

According to the ninth criterion in isolation not part of the invention, control by the rotational resistance control unit 100 to weaken the bend resistance is enabled provided that a state, in which the forward movement amount sensed by the forward movement amount sensing unit 104 is equal to or greater than a forward movement amount threshold value, continues for a predetermined period of time. The spirit of the ninth criterion is the same as that of the eighth criterion. Requiring that a state in which the forward movement amount Lx equal to or greater than a predetermined amount continues to be sensed for a predetermined period of time makes it more certain that the wearer is walking forward ordinarily.

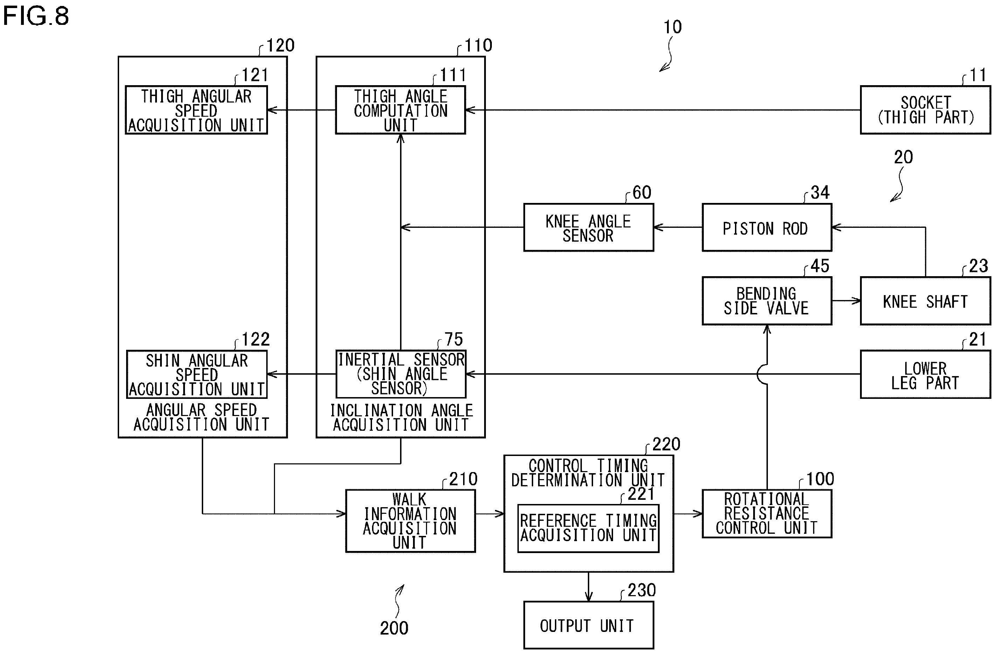

Fig. 8 is a functional block diagram of an adjustment support apparatus 200 for the artificial knee joint 20. The elements corresponding to those of Fig. 6 are denoted by the same symbols, and a description thereof is omitted. The adjustment support apparatus 200 is provided with a walk information acquisition unit 210, a control timing determination unit 220, and an output unit 230 and is implemented in the control unit 50 of the artificial knee joint 20.

The walk information acquisition unit 210 acquires walk information indicating a state of walk of the user of the artificial knee joint 20. Walk information is exemplified by data itself acquired by the knee angle sensor 60, the inertial sensor 75, the thigh angle computation unit 111, the angular speed acquisition unit 120, etc., the posture and motion (speed and acceleration) of the respective parts of the prosthetic limb 10 that can be sensed from the data, the walking mode sensed based on the above items (see the description of the walking mode sensing unit 102 of Fig. 6), the load line (see the description of the load line acquisition unit 103 of Fig. 6), and the forward movement amount (see the description of the forward movement amount sensing unit 104 of Fig. 6). These items of walk information may be acquired in a test walk done by the user wearing the artificial knee joint 20 for adjustment of the artificial knee joint 20 or may be acquired in an ordinary walk of the user wearing the artificial knee joint 20 after the adjustment.

The control timing determination unit 220 determines the timing according to which the rotational resistance control unit 100 weakens the bend resistance of the knee shaft 23, based on the walk information acquired by the walk information acquisition unit 210. More specifically, the control timing determination unit 220 determines the timing to weaken the bend resistance according to the first to ninth criteria described with reference to Fig. 6. For example, the control timing determination unit 220 may, in accordance with the first criterion, determine the point of time when the thigh angle Ψ computed by the thigh angle computation unit 111 makes a transition from positive to negative as the point of time when the rotational resistance control unit 100 weakens the bend resistance of the knee shaft 23. Alternatively, the control timing determination unit 220 may determine the point of time when the thigh angle Ψ decreasing from positive to negative reaches a threshold value set by an adjustment personnel such as a prosthetist as the point of time when the rotational resistance control unit 100 weakens the bend resistance of the knee shaft 23. The threshold value can be set as desired by the adjustment personnel but is preferably set between -3 degrees and -8 degrees. The threshold value may be 0 degrees. It is preferable to define a negative thigh angle Ψ that cannot be formed when the wearer is standing upright ordinarily as the threshold value, because knee instability may occur contrary to the intension of the wearer of the artificial knee joint 20 when the wearer is standing upright with the thigh angle of 0 degrees. Even when the prosthetist has set -4 degrees, the bend resistance of the knee shaft 23 may be weakened at +1 degree in consideration of a control delay of 100 ms in the case of the thigh angular speed of -0.05 deg/ms.

Alternatively, the control timing determination unit 220 may determine the point of time when the absolute value of the negative angular speed of the thigh angle Ψ acquired by the thigh angular speed acquisition unit 121 exceeds a predetermined value as the point of time when the rotational resistance control unit 100 weakens the bend resistance of the knee shaft 23. Alternatively, the control timing determination unit 22 may determine the point of time when the acceleration of the wearer of the knee shaft 20 in the direction of travel exceeds a predetermined acceleration threshold value as the point of time when the rotational resistance control unit 100 weakens the bend resistance of the knee shaft 23. Alternatively, the control timing determination unit 220 may, in accordance with the second criterion, determine the point of time when the knee shaft 23 makes a transition from a point behind the load line to a point in front as the point of time when the rotational resistance control unit 100 weakens the bend resistance of the knee shaft 23. Alternatively, the control timing determination unit 220 may, in accordance with the eighth criterion, determine the point of time when the forward movement amount exceeds the forward movement amount threshold value as the point of time when the rotational resistance control unit 100 weakens the bend resistance of the knee shaft 23.

The control timing determination unit 220 may determine the point of time when the rotational resistance control unit 100 weakens the bend resistance of the knee shaft 23 based on a reference point of time set by the adjustment personnel of the artificial knee joint 20 and acquired by a reference timing acquisition unit 221. More specifically, the control timing determination unit 220 compares the walk information recorded when the reference timing was set by the adjustment personnel such as a prosthetist with the current walk information acquired by the walk information acquisition unit 210 in real time, adds the variation determined by the discrepancy to the reference point of time, and defines the result as the point of time to weaken the bend resistance.

The output unit 230 outputs the timing determined by the control timing determination unit 220 outside. The adjustment personnel such as a prosthetist can adjust the artificial knee joint 20 efficiently for each user, while referring to the timing output in accordance with the actual walk information on the user wearing the artificial knee joint 20.

Described above is an explanation of the present invention based on an embodiment. The embodiment is intended to be illustrative only and it will be understood by those skilled in the art that various modifications to constituting elements and processes could be developed and that such modifications are also within the scope of the present invention.

The functional configuration of the apparatuses described in the embodiment can be realized by hardware resources or software resources or a cooperation of hardware resources and software resources Processors, ROMs, RAMs, and other LSIs can be used as hardware resources. Programs such as operating systems and applications can be used as software resources.

In those the embodiments disclosed in this specification in which a plurality of functions are distributed, some or all of the plurality of functions may be aggregated. Conversely, an aggregation of a plurality of functions may be distributed in part of in the entirety. Regardless of whether functions are aggregated or distributed, the functions may be configured to achieve the purpose of the invention.

The present invention is defined by the appended claims.

## Claims

1. An artificial knee joint (20) comprising:
a thigh joint part (22) provided on a side of a thigh part;
a lower leg part (21) coupled to the thigh joint part (22) and provided to be rotatable around a knee shaft (23);
a thigh part inclination angle acquisition unit that acquires an inclination angle formed by the thigh part relative to a vertical line passing through the knee shaft (23);
a rotational resistance control unit (100) that weakens a rotational resistance of the knee shaft (23) in accordance with a transition from a positive inclination angle formed when the thigh part is inclined rearward relative to the vertical line to a negative inclination angle formed when the thigh part is inclined forward relative to the vertical line, **characterized by** further comprising:
a thigh part inclination angular speed acquisition unit that acquires an angular speed of the inclination angle of the thigh part; and
a delay time acquisition unit (101) that acquires a delay time that elapses until a result of control by the rotational resistance control unit (100) is reflected in the rotational resistance, wherein
when the angular speed of the thigh part is negative, the rotational resistance control unit (100) starts controlling the rotational resistance of the knee shaft (23) to be weaker while the inclination angle of the thigh part is larger than a predetermined threshold value so as to prevent the inclination angle of the thigh part from falling below the threshold value during the delay time.

2. The artificial knee joint (20) according to claim 1, further comprising:
a load line acquisition unit (103) that acquires a load line indicating a direction of load exerted on the lower leg part (21), wherein
the rotational resistance control unit (100) weakens the rotational resistance of the knee shaft (23) before the knee shaft (23) makes a transition from a point behind the load line to a point in front of the load line.

3. The artificial knee joint (20) according to claim 1 or 2, further comprising:
a walking mode sensing unit (102) that senses a walking mode of a user wearing the artificial knee joint (20), and
when the walking mode sensed is a particular walking mode, the rotational resistance control unit (100) does not control the rotational resistance of the knee shaft (23) to be weaker even when the thigh part makes a transition from a positive inclination angle to a negative inclination angle.

4. The artificial knee joint (20) according to any one of claims 1 through 3, further comprising:
an angular speed acquisition unit (120) that acquires an angular speed of the inclination angle formed by at least one of the thigh part and lower leg part (21) relative to the vertical line, wherein
the rotational resistance control unit (100) weakens the rotational resistance of the knee shaft (23) when the angular speed acquired by the angular speed acquisition unit (120) remains negative for a predetermined period of time continuously during a transition of the thigh part from a positive inclination angle to a negative inclination angle.

5. The artificial knee joint (20) according to any one of claims 1 through 4, further comprising:
a knee angle acquisition unit that acquires a knee angle that is a rotational angle of the knee shaft (23), wherein
the rotational resistance control unit (100) weakens the rotational resistance of the knee shaft (23) when the knee angle is equal to or smaller than a predetermined value in a transition of the thigh part from a positive inclination angle to a negative inclination angle.

6. The artificial knee joint (20) according to any one of claims 1 through 5, further comprising:
a knee angle acquisition unit that acquires a knee angle that is a rotational angle of the knee shaft (23); and
a lower leg part inclination angle acquisition unit that acquires an inclination angle formed by the lower leg part (21) relative to the vertical line, wherein
the thigh part inclination angle acquisition unit computes the inclination angle of the thigh part based on the knee angle acquired by the knee angle acquisition unit and the inclination angle of the lower leg part (21) acquired by the lower leg part inclination angle acquisition unit.

7. The artificial knee joint (20) according to any one of claims 1 through 6, further comprising:
a load sensing unit that senses a load exerted on the lower leg part (21), wherein
the rotational resistance control unit (100) does not control the rotational resistance of the knee shaft (23) to be weaker or controls the rotational resistance to be stronger when the inclination angle of the thigh part makes a transition from positive to negative while the load sensing unit does not detect a load.

8. The artificial knee joint (20) according to any one of claims 1 through 7 provided with a thigh joint part (22) provided on a side of a thigh part and with a lower leg part (21) coupled to the thigh joint part (22) and provided to be rotatable around a knee shaft (23), including:
a forward movement amount sensing unit (104) that senses a forward movement amount of a user wearing the artificial knee joint (20); and
a rotational resistance control unit (100) that strengthens a rotational resistance of the knee shaft (23) when the forward movement amount sensed makes a transition from a state in which the forward movement amount is equal to or larger than a predetermined forward movement amount threshold value to a state in which forward movement amount is smaller than the forward movement amount threshold value, or, a rotational resistance control unit (100) that enables controlling a rotational resistance of the knee shaft (23) to be weaker when the forward movement amount sensed is equal to or greater than a predetermined forward movement amount threshold value.

9. The artificial knee joint (20) according to claim 8, further including:
a thigh part inclination angle acquisition unit that acquires an inclination angle formed by the thigh part relative to a vertical line passing through the knee shaft (23), wherein
the forward movement amount sensing unit (104) senses the forward movement amount from the inclination angle of the thigh part.

10. The artificial knee joint (20) according to any one of claims 1 through 9 provided with a thigh joint part (22) provided on a side of a thigh part and with a lower leg part (21) coupled to the thigh joint part (22) and provided to be rotatable around a knee shaft (23), including:
a rotational resistance control unit (100) that weakens a rotational resistance of the knee shaft (23) in accordance with a transition of a user walking and wearing the artificial knee joint (20) from a Stance phase to a Swing phase;
a walk information acquisition unit (210) that acquires walk information indicating a state of walk of the user; and
a control timing determination unit (220) that determines a timing according to which the rotational resistance control unit (100) weakens the rotational resistance of the knee shaft (23), based on the walk information.

11. The artificial knee joint (20) according to claim 10, further including:
an output unit (230) that outputs a point of time determined by the control timing determination unit (220) outside.

12. The artificial knee joint (20) according to claim 10 or 11, wherein
the walk information acquisition unit (210) acquires walk information in a test walk done by the user wearing the artificial knee joint (20) for adjustment of the artificial knee joint (20), and
the control timing determination unit (220) determines the timing according to which the rotational resistance control unit (100) weakens the rotational resistance of the knee shaft (23), based on the walk information acquired in the test walk.

13. The artificial knee joint (20) according to any one of claims 10 through 12, further including:
the walk information includes an inclination angle formed by the thigh part relative to a vertical line passing through the knee shaft (23), or, the walk information includes an angular speed of an inclination angle formed by the thigh part relative to a vertical line passing through the knee shaft (23),
the control timing determination unit (220) determine a point of time when a transition is made from a positive inclination angle formed when the thigh part is inclined rearward relative to the vertical line to a negative inclination angle formed when the thigh part is inclined forward relative to the vertical line as the point of time when the rotational resistance control unit (100) weakens the rotational resistance of the knee shaft (23), or, the control timing determination unit (220) determines a point of time when an absolute value of a negative angular speed of the thigh part exceeds a predetermined value as the point of time when the rotational resistance control unit (100) weakens the rotational resistance of the knee shaft (23).

14. The artificial knee joint (20) according to any one of claims 10 through 13, wherein
the walk information includes a forward movement amount of the user wearing the artificial knee joint (20), and
the control timing determination unit (220) determines a point of time when the forward movement amount exceeds a predetermined forward movement amount threshold value as the point of time when the rotational resistance control unit (100) weakens the rotational resistance of the knee shaft (23).

15. The artificial knee joint (20) according to any one of claims 10 through 14, wherein
the walk information includes an acceleration of the user wearing the artificial knee joint (20) in a direction of travel, and
the control timing determination unit (220) determines a point of time when the acceleration exceeds a predetermined acceleration threshold value as the point of time when the rotational resistance control unit (100) weakens the rotational resistance of the knee shaft (23).

16. A method of controlling an artificial knee joint (20) according to any of claim 1-15, the method including:
acquiring an inclination angle formed by the thigh part relative to a vertical line passing through the knee shaft (23); and
weakening a rotational resistance of the knee shaft (23), by a rotational resistance control unit (100) in accordance with a transition from a positive inclination angle formed when the thigh part is inclined rearward relative to the vertical line to a negative inclination angle formed when the thigh part is inclined forward relative to the vertical line,
**characterized by**
acquiring an angular speed of the inclination angle of the thigh part; and
acquiring a delay time that elapses until a result of control by the rotational resistance control unit (100) is reflected in the rotational resistance, wherein
when the angular speed of the thigh part is negative, the rotational resistance control unit (100) starts controlling the rotational resistance of the knee shaft (23) to be weaker while the inclination angle of the thigh part is larger than a predetermined threshold value so as to prevent the inclination angle of the thigh part from falling below the threshold value during the delay time.

17. A computer program comprising instructions which, when the program is executed by a computer, cause the computer to carry out the steps of the control method of claim 16 for controlling an artificial knee joint (20) according to any of claims 1-15,

## Patentansprüche

1. Ein künstliches Kniegelenk (20), umfassend:
einen Oberschenkelverbindungsteil (22), der auf einer Seite eines Oberschenkelteils vorgesehen ist;
einen Unterschenkelteil (21), der mit dem Oberschenkelverbindungsteil (22) gekoppelt ist und um eine Kniewelle (23) drehbar vorgesehen ist;
eine Oberschenkelteilneigungswinkelerfassungseinheit, die einen Neigungswinkel erfasst, der vom Oberschenkelteil relativ zu einer vertikalen Linie gebildet wird, die durch die Kniewelle (23) verläuft; und
eine Rotationswiderstandssteuereinheit (100), die einen Rotationswiderstand der Kniewelle (23) entsprechend einem Übergang von einem positiven Neigungswinkel, der gebildet wird, wenn der Oberschenkelteil relativ zur vertikalen Linie nach hinten geneigt ist, zu einem negativen Neigungswinkel abschwächt, der gebildet wird, wenn der Oberschenkelteil relativ zur vertikalen Linie nach vorne geneigt ist, **gekennzeichnet dadurch, dass** es weiterhin umfasst:
eine Oberschenkelteilneigungswinkelgeschwindigkeiterfassungseinheit, die eine Winkelgeschwindigkeit des Neigungswinkels des Oberschenkelteils erfasst; und
eine Verzögerungszeiterfassungseinheit (101), die eine Verzögerungszeit erfasst, die verstreicht, bis ein Ergebnis der Steuerung durch die Rotationswiderstandssteuereinheit (100) im Rotationswiderstand reflektiert ist, wobei
wenn die Winkelgeschwindigkeit des Oberschenkelteils negativ ist, beginnt die Rotationswiderstandssteuereinheit (100) den Rotationswiderstand der Kniewelle (23) so zu steuern, dass er schwächer wird, während der Neigungswinkel des Oberschenkelteils größer als ein vorgegebener Schwellenwert ist, um zu verhindern, dass der Neigungswinkel des Oberschenkelteils während der Verzögerungszeit unter den Schwellenwert fällt.

2. Das künstliche Kniegelenk (20) nach Anspruch 1, weiterhin umfassend:
eine Lastlinienerfassungseinheit (103), die eine Lastlinie erfasst, die eine Richtung der auf den Unterschenkelteil (21) ausgeübten Last anzeigt, wobei
die Rotationswiderstandssteuereinheit (100) den Rotationswiderstand der Kniewelle (23) abschwächt, bevor die Kniewelle (23) einen Übergang von einem Punkt hinter der Lastlinie zu einem Punkt vor der Lastlinie durchführt.

3. Das künstliche Kniegelenk (20) gemäß Anspruch 1 oder 2, weiterhin umfassend:
eine Gehmoduserfassungseinheit (102), die einen Gehmodus eines Benutzers erkennt, der das künstliche Kniegelenk (20) trägt, und
wenn der erfasste Gehmodus ein bestimmter Gehmodus ist, steuert die Rotationswiderstandssteuereinheit (100) den Rotationswiderstand der Kniewelle (23) nicht so, dass er schwächer wird, selbst wenn der Oberschenkelteil einen Übergang von einem positiven Neigungswinkel zu einem negativen Neigungswinkel durchführt.

4. Das künstliche Kniegelenk (20) gemäß irgendeinem der Ansprüche 1 bis 3, weiterhin umfassend:
eine Winkelgeschwindigkeitserfassungseinheit (120), die eine Winkelgeschwindigkeit des Neigungswinkels erfasst, der von zumindest einem von dem Oberschenkelteil und Unterschenkelteil (21) relativ zur vertikalen Linie gebildet wird, wobei
die Rotationswiderstandssteuereinheit (100) den Rotationswiderstand der Kniewelle (23) schwächt, wenn die von der Winkelgeschwindigkeitserfassungseinheit (120) erfasste Winkelgeschwindigkeit während eines Übergangs des Oberschenkelteils von einem positiven Neigungswinkel zu einem negativen Neigungswinkel für eine vorgegebene Zeitperiode kontinuierlich negativ bleibt.

5. Das künstliche Kniegelenk (20) gemäß irgendeinem der Ansprüche 1 bis 4, weiterhin umfassend:
eine Kniewinkelerfassungseinheit, die einen Kniewinkel erfasst, der ein Rotationswinkel der Kniewelle (23) ist, wobei
die Rotationswiderstandssteuereinheit (100) den Rotationswiderstand der Kniewelle (23) schwächt, wenn der Kniewinkel bei einem Übergang des Oberschenkelteils von einem positiven Neigungswinkel zu einem negativen Neigungswinkel gleich oder kleiner als ein vorgegebener Wert ist.

6. Das künstliche Kniegelenk (20) gemäß irgendeinem der Ansprüche 1 bis 5, ferner umfassend:
eine Kniewinkelerfassungseinheit, die einen Kniewinkel erfasst, der ein Rotationswinkel der Kniewelle (23) ist; und
eine Unterschenkelteilneigungswinkelerfassungseinheit, die einen Neigungswinkel erfasst, der durch das Unterschenkelteil (21) relativ zur vertikalen Linie gebildet wird, wobei
die Oberschenkelteilneigungswinkelerfassungseinheit den Neigungswinkel des Oberschenkelteils auf Grundlage des von der Kniewinkelerfassungseinheit erfassten Kniewinkels und des von der Unterschenkelteilneigungswinkelerfassungseinheit erfassten Neigungswinkels des Unterschenkelteils (21).

7. Das künstliche Kniegelenk (20) gemäß irgendeinem der Ansprüche 1 bis 6, weiterhin umfassend:
eine Lasterfassungseinheit, die eine auf den Unterschenkelteil (21) ausgeübte Last erfasst, wobei
die Rotationswiderstandssteuereinheit (100) den Rotationswiderstand der Kniewelle (23) nicht so steuert, dass er schwächer wird, oder den Rotationswiderstand so steuert, dass er stärker wird, wenn der Neigungswinkel des Oberschenkelteils einen Übergang von positiv zu negativ durchführt, während die Lasterfassungseinheit keine Last detektiert.

8. Das künstliche Kniegelenk (20) gemäß irgendeinem der Ansprüche 1 bis 7, das mit einem auf einer Seite eines Oberschenkelteils vorgesehenen Oberschenkelverbindungsteil (22) und mit einem mit dem Oberschenkelverbindungsteil (22) gekoppelten und um eine Kniewelle (23) drehbar vorgesehenen Unterschenkelteil (21) versehen ist, enthaltend:
eine Vorwärtsbewegungsbetragserfassungseinheit (104), die einen Vorwärtsbewegungsbetrag eines Benutzers erfasst, der das künstliche Kniegelenk (20) trägt; und
eine Rotationswiderstandssteuereinheit (100), die einen Rotationswiderstand der Kniewelle (23) stärkt, wenn der erfasste Vorwärtsbewegungsbetrag einen Übergang von einem Zustand, in dem der Vorwärtsbewegungsbetrag gleich oder größer als ein vorgegebener Vorwärtsbewegungsbetragsschwellenwert ist, zu einem Zustand durchführt, in dem der Vorwärtsbewegungsbetrag kleiner als der Vorwärtsbewegungsbetragsschwellenwert ist, oder eine Rotationswiderstandssteuereinheit (100), die eine Steuerung eines Rotationswiderstands der Kniewelle (23) so ermöglicht, dass er schwächer wird, wenn der erfasste Vorwärtsbewegungsbetrag gleich oder größer als ein vorgegebener Vorwärtsbewegungsbetragsschwellenwert ist.

9. Das künstliche Kniegelenk (20) gemäß Anspruch 8, ferner enthaltend:
eine Oberschenkelteilneigungswinkelerfassungseinheit, die einen Neigungswinkel erfasst, der durch den Oberschenkelteil relativ zu einer vertikalen Linie gebildet wird, die durch die Kniewelle (23) verläuft, wobei
die Vorwärtsbewegungsbetragserfassungseinheit (104) den Vorwärtsbewegungsbetrag von dem Neigungswinkel des Oberschenkelteils erfasst.

10. Das künstliche Kniegelenk (20) gemäß irgendeinem der Ansprüche 1 bis 9, das mit einem auf einer Seite eines Oberschenkelteils vorgesehenen Oberschenkelverbindungsteil (22) und mit einem mit dem Oberschenkelverbindungsteil (22) gekoppelten und um eine Kniewelle (23) drehbar vorgesehenen Unterschenkelteil (21) versehen ist, enthaltend:
eine Rotationswiderstandssteuereinheit (100), die einen Rotationswiderstand des Kniewelle (23) entsprechend einem Übergang eines gehenden und das künstliche Kniegelenk (20) tragenden Benutzers von einer Standphase zu einer Schwungphase schwächt;
eine Gehinformationserfassungseinheit (210), die Gehinformation erfasst, die einen Gehzustand des Benutzers anzeigt; und
eine Steuerzeitpunktbestimmungseinheit (220), die einen Zeitpunkt bestimmt, gemäß dem die Rotationswiderstandssteuereinheit (100) den Rotationswiderstand der Kniewelle (23) schwächt, basierend auf der Gehinformation.

11. Das künstliche Kniegelenk (20) gemäß Anspruch 10, das weiterhin enthält:
eine Ausgabeeinheit (230), die einen von der Steuerzeitpunktbestimmungseinheit (220) bestimmten Zeitpunkt nach außen ausgibt.

12. Das künstliche Kniegelenk (20) nach Anspruch 10 oder 11, wobei
die Gehinformationserfassungseinheit (210) Gehinformation bei einem Testgang, der durch den Benutzer, der das künstliche Kniegelenk (20) trägt, durchgeführt wird, zur Einstellung des künstlichen Kniegelenks (20) erfasst, und
die Steuerzeitpunktbestimmungseinheit (220) den Zeitpunkt bestimmt, gemäß dem die Rotationswiderstandssteuereinheit (100) den Rotationswiderstand der Kniewelle (23) schwächt, basierend auf der beim Testgang erfassten Gehinformation.

13. Das künstliche Kniegelenk (20) gemäß irgendeinem der Ansprüche 10 bis 12, ferner enthaltend:
die Gehinformation einen Neigungswinkel enthält, der vom Oberschenkelteil relativ zu einer vertikalen Linie gebildet wird, die durch die Kniewelle (23) verläuft, oder die Gehinformation eine Winkelgeschwindigkeit eines Neigungswinkels enthält, der vom Oberschenkelteil relativ zu einer vertikalen Linie gebildet wird, die durch die Kniewelle (23) verläuft,
die Steuerzeitpunktbestimmungseinheit (220) einen Zeitpunkt, zu dem ein Übergang von einem positiven Neigungswinkel, der gebildet wird, wenn der Oberschenkelteil relativ zur vertikalen Linie nach hinten geneigt ist, zu einem negativen Neigungswinkel, der gebildet wird, wenn der Oberschenkelteil relativ zur vertikalen Linie nach vorne geneigt ist, durchgeführt wird, als den Zeitpunkt bestimmt, zu dem die Rotationswiderstandssteuereinheit (100) den Rotationswiderstand der Kniewelle (23) schwächt, oder die Steuerzeitpunktbestimmungseinheit (220) einen Zeitpunkt bestimmt, zu dem ein Absolutwert einer negativen Winkelgeschwindigkeit des Oberschenkelteils einen vorgegebenen Wert überschreitet, als den Zeitpunkt bestimmt, zu dem die Rotationswiderstandssteuereinheit (100) den Rotationswiderstand der Kniewelle (23) schwächt.

14. Das künstliche Kniegelenk (20) nach irgendeinem der Ansprüche 10 bis 13, wobei
die Gehinformation einen Vorwärtsbewegungsbetrag des Benutzers enthält, der das künstliche Kniegelenk (20) trägt, und
die Steuerzeitpunktbestimmungseinheit (220) einen Zeitpunkt, zu dem der Vorwärtsbewegungsbetrag einen vorgegebenen Vorwärtsbewegungsbetragschwellenwert überschreitet, als den Zeitpunkt bestimmt, zu dem die Rotationswiderstandssteuereinheit (100) den Rotationswiderstand der Kniewelle (23) schwächt.

15. Das künstliche Kniegelenk (20) gemäß irgendeinem der Ansprüche 10 bis 14, wobei
die Gehinformation eine Beschleunigung des das künstliche Kniegelenk (20) tragenden Benutzers in einer Fortbewegungsrichtung enthält, und
die Steuerzeitpunktbestimmungseinheit (220) einen Zeitpunkt, zu dem die Beschleunigung einen vorgegebenen Beschleunigungsschwellenwert überschreitet, als den Zeitpunkt bestimmt, zu dem die Rotationswiderstandssteuereinheit (100) den Rotationswiderstand der Kniewelle (23) schwächt.

16. Ein Verfahren zum Steuern eines künstlichen Kniegelenks (20) gemäß irgendeinem der Ansprüche 1 bis 15, wobei das Verfahren enthält:
Erfassen eines Neigungswinkels, der durch den Oberschenkelteil relativ zu einer vertikalen Linie gebildet wird, die durch die Kniewelle (23) verläuft; und
Abschwächen eines Rotationswiderstandes der Kniewelle (23) entsprechend einem Übergang von einem positiven Neigungswinkel, der gebildet wird, wenn der Oberschenkelteil relativ zur vertikalen Linie nach hinten geneigt ist, zu einem negativen Neigungswinkel, der gebildet wird, wenn der Oberschenkelteil relativ zur vertikalen Linie nach vorne geneigt ist, **gekennzeichnet durch**
Erfassen einer Winkelgeschwindigkeit des Neigungswinkels des Oberschenkelteils; und
Erfassen einer Verzögerungszeit, die verstreicht, bis ein Ergebnis der Steuerung durch die Rotationswiderstandssteuereinheit (100) im Rotationswiderstand reflektiert ist, wobei
wenn die Winkelgeschwindigkeit des Oberschenkelteils negativ ist, die Rotationswiderstandssteuereinheit (100) beginnt, den Rotationswiderstand der Kniewelle (23) so zu steuern, dass er schwächer wird, während der Neigungswinkel des Oberschenkelteils größer als ein vorgegebener Schwellenwert ist, um zu verhindern, dass der Neigungswinkel des Oberschenkelteils während der Verzögerungszeit unter den Schwellenwert fäll

17. Ein Computerprogramm, das Anweisungen umfasst, die, wenn das Programm durch einen Computer ausgeführt wird, den Computer veranlassen, die Schritte des Steuerverfahrens von Anspruch 16 zum Steuern eines künstlichen Kniegelenks (20) gemäß irgendeinem der Ansprüche 1 bis 15 auszuführen.

## Revendications

1. Articulation artificielle du genou (20) comprenant :
une partie d'articulation de cuisse (22) pourvue sur un côté d'une partie de cuisse ;
une partie de jambe inférieure (21) couplée à la partie d'articulation de cuisse (22) et pourvue pour pouvoir tourner autour d'un axe de genou (23) ;
une unité d'acquisition d'angle d'inclinaison de partie de cuisse qui acquiert un angle d'inclinaison formé par la partie de cuisse par rapport à une ligne verticale passant par l'axe de genou (23) ;
une unité de contrôle de résistance à la rotation (100) qui affaiblit une résistance à la rotation de l'axe de genou (23) conformément à une transition d'un angle d'inclinaison positif formé quand la partie de cuisse est inclinée vers l'arrière par rapport à la ligne verticale à un angle d'inclinaison négatif formé lorsque la partie de cuisse est inclinée vers l'avant par rapport à la ligne verticale, **caractérisée en ce qu'**elle comprend en outre :
une unité d'acquisition de vitesse angulaire d'inclinaison de la partie de cuisse qui acquiert une vitesse angulaire de l'angle d'inclinaison de la partie de cuisse ; et
une unité d'acquisition de temps de retard (101) qui acquiert un temps de retard écoulé jusqu'à ce qu'un résultat de contrôle par l'unité de contrôle de résistance à la rotation (100) soit reflété dans la résistance à la rotation, dans laquelle
quand la vitesse angulaire de la partie de cuisse est négative, l'unité de contrôle de résistance à la rotation (100) commence à contrôler la résistance à la rotation de l'axe de genou (23) pour qu'elle soit plus faible quand l'angle d'inclinaison de la partie de cuisse est supérieur à une valeur seuil prédéterminée de manière à empêcher que l'angle d'inclinaison de la partie de cuisse tombe sous la valeur seuil pendant le temps de retard.

2. Articulation artificielle du genou (20) selon la revendication 1, comprenant en outre :
une unité d'acquisition de ligne de charge (103) qui acquiert une ligne de charge indiquant une direction de charge exercée sur la partie de jambe inférieure (21), dans laquelle
l'unité de contrôle de résistance à la rotation (100) affaiblit la résistance à la rotation de l'axe de genou (23) avant que l'axe de genou (23) n'effectue une transition d'un point derrière la ligne de charge à un point devant la ligne de charge.

3. Articulation artificielle du genou (20) selon la revendication 1 ou 2, comprenant en outre :
une unité de détection de mode de marche (102) qui détecte un mode de marche d'un utilisateur portant l'articulation artificielle du genou (20), et
quand le mode de marche détecté est un mode de marche particulier, l'unité de contrôle de résistance à la rotation (100) ne contrôle pas la résistance à la rotation de l'axe de genou (23) pour qu'elle soit plus faible même quand la partie de cuisse effectue une transition d'un angle d'inclinaison positif à un angle d'inclinaison négatif.

4. Articulation artificielle du genou (20) selon l'une quelconque des revendications 1 à 3, comprenant en outre :
une unité d'acquisition de vitesse angulaire (120) qui acquiert une vitesse angulaire de l'angle d'inclinaison formé par au moins une partie parmi la partie de cuisse et la partie de jambe inférieure (21) par rapport à la ligne verticale, dans laquelle
l'unité de contrôle de résistance à la rotation (100) affaiblit la résistance à la rotation de l'axe de genou (23) quand la vitesse angulaire acquise par l'unité d'acquisition de vitesse angulaire (120) reste continuellement négative pendant une période prédéterminée pendant une transition de la partie de cuisse d'un angle d'inclinaison positif à un angle d'inclinaison négatif.

5. Articulation artificielle du genou (20) selon l'une quelconque des revendications 1 à 4, comprenant en outre :
une unité d'acquisition d'angle du genou qui acquiert un angle du genou qui est un angle de rotation de l'axe de genou (23), dans laquelle
l'unité de contrôle de résistance à la rotation (100) affaiblit la résistance à la rotation de l'axe de genou (23) quand l'angle du genou est inférieur ou égal à une valeur prédéterminée lors d'une transition de la partie de cuisse d'un angle d'inclinaison positif à un angle d'inclinaison négatif.

6. Articulation artificielle du genou (20) selon l'une quelconque des revendications 1 à 5, comprenant en outre :
une unité d'acquisition d'angle du genou qui acquiert un angle du genou qui est un angle de rotation de l'axe de genou (23) ; et
une unité d'acquisition d'angle d'inclinaison de la partie de jambe inférieure qui acquiert un angle d'inclinaison formé par la partie de jambe inférieure (21) par rapport à la ligne verticale, dans laquelle
l'unité d'acquisition d'angle d'inclinaison de partie de cuisse calcule l'angle d'inclinaison de la partie de cuisse sur la base de l'angle du genou acquis par l'unité d'acquisition de l'angle du genou et de l'angle d'inclinaison de la partie de jambe inférieure (21) acquis par l'unité d'acquisition de l'angle d'inclinaison de la partie de jambe inférieure.

7. Articulation artificielle du genou (20) selon l'une quelconque des revendications 1 à 6, comprenant en outre :
une unité de détection de charge qui détecte une charge exercée sur la partie de jambe inférieure (21), dans laquelle
l'unité de contrôle de résistance à la rotation (100) ne contrôle pas la résistance à la rotation de l'axe de genou (23) pour qu'elle soit plus faible, ou contrôle la résistance à la rotation pour qu'elle soit plus forte, quand l'angle d'inclinaison de la partie de cuisse effectue une transition du positif au négatif alors que l'unité de détection de charge ne détecte pas de charge.

8. Articulation artificielle du genou (20) selon l'une quelconque des revendications 1 à 7, pourvue d'une partie d'articulation de cuisse (22) agencée sur un côté d'une partie de cuisse et d'une partie de jambe inférieure (21) couplée à la partie d'articulation de cuisse (22) et pourvue pour pouvoir tourner autour d'un axe de genou (23), comprenant :
une unité de détection de quantité de déplacement vers l'avant (104) qui détecte une quantité de déplacement vers l'avant d'un utilisateur portant l'articulation artificielle du genou (20) ; et
une unité de contrôle de résistance à la rotation (100) qui renforce une résistance à la rotation de l'axe de genou (23) quand la quantité de déplacement vers l'avant détectée effectue une transition d'un état dans lequel la quantité de déplacement vers l'avant est supérieure ou égale à une valeur seuil de quantité de déplacement vers l'avant prédéterminée à un état dans lequel la quantité de déplacement vers l'avant est inférieure à la valeur seuil de quantité de déplacement vers l'avant, ou une unité de contrôle de résistance à la rotation (100) qui permet de contrôler une résistance à la rotation de l'axe de genou (23) pour qu'elle soit plus faible quand la quantité de déplacement vers l'avant détectée est supérieure ou égale à une valeur seuil de quantité de déplacement vers l'avant prédéterminée.

9. Articulation artificielle du genou (20) selon la revendication 8, comprenant en outre :
une unité d'acquisition d'angle d'inclinaison de partie de cuisse qui acquiert un angle d'inclinaison formé par la partie de cuisse par rapport à une ligne verticale passant par l'axe de genou (23), dans laquelle
l'unité de détection de quantité de déplacement vers l'avant (104) détecte la quantité de déplacement vers l'avant à partir de l'angle d'inclinaison de la partie de cuisse.

10. Articulation artificielle du genou (20) selon l'une quelconque des revendications 1 à 9, pourvue d'une partie d'articulation de cuisse (22) agencée sur un côté d'une partie de cuisse et d'une partie de jambe inférieure (21) couplée à la partie d'articulation de cuisse (22) et pourvue pour pouvoir tourner autour d'un axe de genou (23), comprenant :
une unité de contrôle de résistance à la rotation (100) qui affaiblit une résistance à la rotation de l'axe de genou (23) conformément à une transition d'un utilisateur marchant et portant l'articulation artificielle du genou (20) d'une phase de posture à une phase de balancement ;
une unité d'acquisition d'information de marche (210) qui acquiert de l'information de marche indiquant un état de marche de l'utilisateur ; et
une unité de détermination de synchronisation de contrôle (220) qui détermine une synchronisation selon laquelle l'unité de contrôle de résistance à la rotation (100) affaiblit la résistance à la rotation de l'axe de genou (23), sur la base de l'information de marche.

11. Articulation artificielle du genou (20) selon la revendication 10, comprenant en outre :
une unité de sortie (230) qui sort vers l'extérieur un point temporel déterminé par l'unité de détermination de synchronisation de contrôle (220).

12. Articulation artificielle du genou (20) selon la revendication 10 ou 11, dans laquelle
l'unité d'acquisition d'information de marche (210) acquiert de l'information de marche lors d'une marche d'essai effectuée par l'utilisateur portant l'articulation artificielle du genou (20) pour le réglage de l'articulation artificielle du genou (20), et
l'unité de détermination de synchronisation de contrôle (220) détermine la synchronisation selon lequel l'unité de contrôle de résistance à la rotation (100) affaiblit la résistance à la rotation de l'axe de genou (23), sur la base de l'information de marche acquise lors de la marche d'essai.

13. Articulation artificielle du genou (20) selon l'une quelconque des revendications 10 à 12, dans laquelle en outre :
l'information de marche comprend un angle d'inclinaison formé par la partie de cuisse par rapport à une ligne verticale passant par l'axe de genou (23), ou l'information de marche comprend une vitesse angulaire d'un angle d'inclinaison formé par la partie de cuisse par rapport à une ligne verticale passant par l'axe de genou (23),
l'unité de détermination de synchronisation de contrôle (220) détermine un point temporel où une transition est effectuée d'un angle d'inclinaison positif formé quand la partie de cuisse est inclinée vers l'arrière par rapport à la ligne verticale à un angle d'inclinaison négatif formé quand la partie de cuisse est inclinée vers l'avant par rapport à la ligne verticale comme point temporel où l'unité de contrôle de résistance à la rotation (100) affaiblit la résistance à la rotation de l'axe de genou (23), ou l'unité de détermination de synchronisation de contrôle (220) détermine un point temporel où une valeur absolue de la vitesse angulaire négative de la partie de cuisse dépasse une valeur prédéterminée comme point temporel où l'unité de contrôle de résistance à la rotation (100) affaiblit la résistance à la rotation de l'axe de genou (23) .

14. Articulation artificielle du genou (20) selon l'une quelconque des revendications 10 à 13, dans laquelle
l'information de marche comprend une quantité de déplacement vers l'avant de l'utilisateur portant l'articulation artificielle du genou (20), et
l'unité de détermination de synchronisation de contrôle (220) détermine un point temporel où la quantité de déplacement vers l'avant dépasse une valeur seuil de quantité de déplacement vers l'avant prédéterminée comme point temporel où l'unité de contrôle de résistance à la rotation (100) affaiblit la résistance à la rotation de l'axe de genou (23).

15. Articulation artificielle du genou (20) selon l'une quelconque des revendications 10 à 14, dans laquelle
l'information de marche comprend une accélération de l'utilisateur portant l'articulation artificielle du genou (20) dans une direction de déplacement, et
l'unité de détermination de synchronisation de contrôle (220) détermine un point temporel où l'accélération dépasse une valeur seuil d'accélération prédéterminée comme point temporel où l'unité de contrôle de résistance à la rotation (100) affaiblit la résistance à la rotation de l'axe de genou (23).

16. Procédé de contrôle d'une articulation artificielle du genou (20) selon l'une quelconque des revendications 1 à 15, le procédé comprenant :
l'acquisition d'un angle d'inclinaison formé par la partie de cuisse par rapport à une ligne verticale passant par l'axe de genou (23) ; et
l'affaiblissement d'une résistance à la rotation de l'axe de genou (23) par une unité de contrôle de résistance à la rotation (100) conformément à une transition d'un angle d'inclinaison positif formé quand la partie de cuisse est inclinée vers l'arrière par rapport à la ligne verticale à un angle d'inclinaison négatif formé quand la partie de cuisse est inclinée vers l'avant par rapport à la ligne verticale,
**caractérisé par**
l'acquisition d'une vitesse angulaire de l'angle d'inclinaison de la partie de cuisse ; et
l'acquisition d'un temps de retard qui s'écoule jusqu'à ce qu'un résultat de contrôle par l'unité de contrôle de résistance à la rotation (100) soit reflété dans la résistance à la rotation, dans lequel
quand la vitesse angulaire de la partie de cuisse est négative, l'unité de contrôle de résistance à la rotation (100) commence à contrôler la résistance à la rotation de l'axe de genou (23) pour qu'elle soit plus faible quand l'angle d'inclinaison de la partie de cuisse est supérieur à une valeur seuil prédéterminée de manière à empêcher que l'angle d'inclinaison de la partie de cuisse tombe sous la valeur seuil pendant le temps de retard.

17. Programme informatique comprenant des instructions qui, quand le programme est exécuté par un ordinateur, commandent à l'ordinateur de mettre en œuvre les étapes du procédé de contrôle selon la revendication 16 pour contrôler une articulation artificielle du genou (20) selon l'une quelconque des revendications 1 à 15.
